# EUROPEAN PATENT APPLICATION

(11) **EP 3 081 160 A1**
(43) Date of publication of application: **19.10.2016**
(21) Application number: 13899021.3
(22) Date of filing: 10.12.2013
(51) Int. Cl.: A61B 5/103

(54) **SYSTEM FOR ASSISTING IN BALANCING BODY WEIGHT**

(71) Applicant: Operacion Sonrie S.L., 39009 Santander (ES)
(72) Inventor: CATALINA PEÑA, Antonio, E-39009 Santander (ES)
(74) Representative: Ezcurra Zufia, Maria Antonia
(86) International application number: PCT/ES2013/070861
(87) International publication number: WO 2015/086862

(57) **Abstract**

System for assisting in balancing body weight containing a footwear item and a smartphone or similar, where the footwear item comprises means for detecting the proportion of the weight bearing on the front part of the foot and on the rear part of the foot, preferably load sensors (1 and 2), a printed circuit board (4) for controlling and transmitting / receiving signals, a power supply battery (5) for powering the elements installed in the footwear item and vibrating motors (3) positioned in the front part of the foot and in front of the front load sensors (3). An application is installed in the smartphone and used to control the operation of the system and consequently, the footwear item and the exercises to be performed thereon.

## Description

### OBJECT OF THE INVENTION

As the title indicates, the object of this invention is a system for assisting in balancing body weight, including a footwear item that allows assistance in balancing body weight.

The present invention is characterised by the special construction characteristics of the footwear item, its component elements, the synergy achieved by the union of the functions of the component elements, and the possibility of integrating these elements within a system that detects and corrects positional defects in the human spinal column.

This invention therefore falls within both the area of footwear and of those systems that help detect defects in positioning of the human spinal column of the human body, particular when standing and walking.

### BACKGROUND TO THE INVENTION

According to neurosurgeons dealing with the spinal column and other experts in this area, problems related to the spinal column have become a major challenge, affecting many people in our society, independently of race, sex, age or occupation, with an important impact on health and on individual and business productivity.

The main symptoms are generally related to cervical and/or lumbar pain, although other resulting effects often occur at the same time. These may include weakness in the legs, headaches, insomnia, loss of attention, depression, etc.

Lumbar pain is one of the most frequent complaints raised with medical consultants. Its incidence is increasing in our population, especially among children and young people, a situation that has not been seen for decades. The main causes of these pains and their associated lesions are changes in our lifestyle, with people spending more time seated, lack of physical activity, obesity and in particular, bad posture and misuse of the body when performing a range of everyday actions,.

As a result, problems with the spinal column are ever more common in our society. These include painful lumbar and cervical disc herniation, headaches and neck pain, etc., all of which have become a cause for reduced business performance and very common grounds for sick leave.

Statistics show that everyone suffers an episode of low back pain at some point in their lives and between 5% and 25% of the population suffer one LBP per year. Cervical pain is one of the main causes of sick leave, with LBP leading to the loss of an average of 6,000,000 working days per year at a very high cost. In the European Union around 67 million people are estimated to suffer from this affliction.

Over the last three years, 89% of Spaniards have suffered one such incident as a result of the use of laptop computers, tablets, latest-generation video consoles, mobile phones and iPods. The main focus of the discomfort tends to be in the back and neck muscles, but pain is also common in fingers, elbows, eyes and ears.

Given that the great majority of back pains and conditions are a consequence of incorrect distribution of weight on the feet, the purpose of the invention is to develop a footwear item that will assist in detecting and correcting the distribution of body weight on the sole of the feet. The characteristics of this item of footwear is described below and set out in essence in Claim One.

### DESCRIPTION OF THE INVENTION

The object of this invention is, on the one hand, an item of footwear for assisting in balancing body weight and an electronic system which includes the footwear item and which co-operates with the footwear item in assisting in balancing body weight on the sole of the feet.

The footwear item is capable of indicating when the weight is badly distributed and issuing a stimulus that will correct the posture in a natural fashion.

It is an item of footwear that assists in balancing body weight, making it lighter for performing any activity. It assists in correcting posture and preventing pains and injury in the back, mainly in the lower back, since with its use the person ceases to bear all their weight in this area of the spine, as approximately 90% of the population unconsciously do.

Body weight is normally placed slightly to the rear, being supported more in the area of the heel, resulting in locking of the knees because of a false notion that they provide greater resistance and better support the weight, when in fact the opposite is true, creating more tension especially in the lower back.

This causes a constant bearing of the body weight in the lumbar area, which in turn causes the heel to hit the ground when walking, resulting in an unconsciously "military" form of walking, with the rear part of the body leaning backward and the heels hitting the ground.

When walking, this causes more impact and tension in the lower back. This effect, which is almost imperceptible if careful attention is not paid, results in more tension, pain and different injuries over time as a result of the friction and pressure of the vertebrae, as well as wear on the intervertebral discs, because the spaces between the vertebrae in the lumbar area are more tightly closed, causing impact and friction between them, pinching of the nerve and deterioration of the discs.

The purpose of this footwear item is to detect when the body weight is inclined slightly to the rear, bearing on the lumbar area of the back. This measurement is performed with various weight sensors positioned in the base of the footwear item which comes in contact with the foot, two in the front and two in the rear of the sole of the footwear item. This information is gathered in an integrated circuit board or in a printed circuit board, and sent by Bluetooth or other means of wireless communication, to an application installed in a mobile terminal which will constitute the command centre.

The integrated circuit board is configured to gather precise information on how the body weight is distributed in the footstep. When the body weight exceeds a given threshold of imbalance, with options ranging from 60% to 70% of the weight bearing on the rear, an alarm is activated which will be sent by a Bluetooth device with this information to the application installed in the mobile.

Once this information has been gathered and the alarm has been sent to the application on the mobile, the person will be able to see, on a screen in the application, how their body weight is distributed across their footstep. This information includes both the proportion of the weight bearing on the front and rear, and the proportion of the weight bearing on each foot.

The application shows these data as percentages and displays a graph depicting a body with the corresponding degree of imbalance based on the data received from the footwear item, showing whether the weight is bearing towards the rear or to one side. The user can see whether his or her posture is imbalanced and in tension by seeing the degree of inclination on the display.

The user can then decide whether he/she wishes to correct the posture using any of the exercises recommended by the application. If so, he/she then begins an exercise whereby he/she can correct this posture by indicating the correct position to the body by means of posture training and re-education.

The application indicates the requirements of time and space for performing the exercise, namely about 10 minutes in an area where the user will not be disturbed and can pay full attention to working on his/her posture. The user is then asked whether he/she wishes to begin the exercise at that time or postpone it until later.

The application offers different options for exercises. When the user decides to commence the necessary posture re-education, the application activates a voice that explains the necessary guidelines for beginning the exercise, and tells the user to sit in a comfortable position. A picture is displayed showing how the user should sit in the chair and how to position feet, legs, spine and head, an essential and generic feature of all exercises whereby the person will learn to perform on his/her own with use and practice of the exercises.

Once the user has assumed the right posture to perform the exercise, the audio will direct his/her attention to different parts of their body, in order to perceive ever more precisely what is happening in each one. This is one part of the training proposed by the footwear item, whereby users gradually become aware of the feeling of their body, what is happening in it and how it performs different things and they become conscious of the posture they adopt at different times of the day. By becoming aware of what they are doing wrong, they can stop doing it and correct their posture.

The user is now prepared for the next step, stimulation by vibration to correct loading of the weight in a natural fashion. The footwear item has four vibrating motors in the front part of the foot, which in one possible preferential embodiment, will be in front of the front weight sensor, in the area known as the ball of the foot.

These motors are in contact with the sole of the foot. Via an audio, the application directs the user's attention to feel this stimulus precisely and the motors begin to vibrate one after another in 2-minute sequences. First the motor in the left-hand side of the footwear item begins to vibrate, followed by the one on the right-hand side, and then all four motors at the same time for a further two minutes, up to 10 minutes. The purpose is to stimulate the entire rear area of the sole of the foot, and to focus the user's attention on that area.

The aim of the stimulation is to make users feel the front part of the foot as being wider, helping them to become aware of this part and to realise that the weight can be better distributed on the sole, and less concentrated on the heel, and that the weight can be distributed more to this wider area of the foot. Once the sequential stimulation exercise is completed, the distribution of the weight is again measured and shown on the graph to see whether it is indeed better balanced and to what extent.

This is the function of the footwear item, to measure and locate badly proportioned bearing of body weight, based on the pressure of the footstep, and to stimulate the front of the foot so that by feeling that the foot and footstep are larger, this sensation provides more stability and helps users to balance the weight of their body naturally.

By way of the application for the mobile, etc. and through different practical exercises, this footwear teaches users to pay attention, feel, relax and reduce stress by practising different 10-minute exercises. It educates the body to learn to distribute the weight properly in order to prevent injury to the lower back area. It favours lighter standing and walking when users begin to place more weight on the front part of the foot rather than the heel, reduces fatigue when walking, helps increase the user's confidence, concentration, attention and general wellbeing.

The function of this product is to remedy and prevent different problems and ailments currently suffered by a large proportion of society, sooner or later, to a greater or lesser extent. It is thus intended to provide a practical solution to pains and problems caused by bad postures and misuse of body weight, reducing fatigue and pain in the lower back, head, legs, etc. as well as stress and depression.

### DESCRIPTION OF FIGURES

To complete the description made herein and in order better to aid understanding of the characteristics of the invention, according to a preferential example of a practical embodiment thereof, this description is accompanied by a set of drawings showing as follows in indicative, but not limitative terms.
Figure 1 shows a schematic depiction of the sole of an item of footwear showing the means for detecting and assisting in balancing body weight in the footwear.

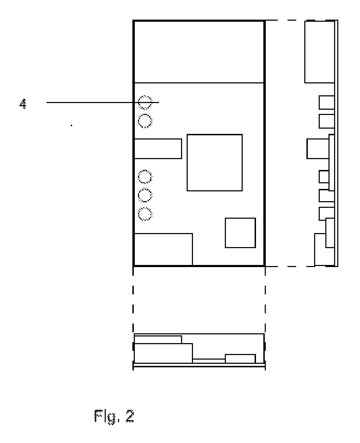

Figure 2 shows a depiction of the printed circuit board used in the footwear item.

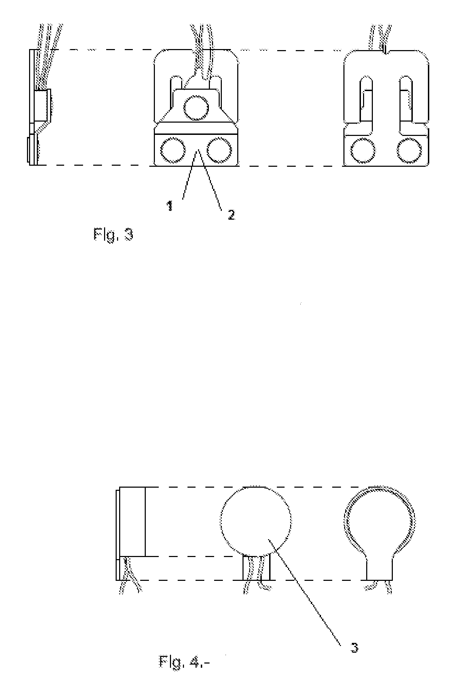

Figure 3 shows a depiction of a load sensor.
Figure 4 shows different views of one of the vibrating motors.

### PREFERRED EMBODIMENT OF THE INVENTION.

In view of the figures one form of preferred embodiment of the proposed invention is described below.

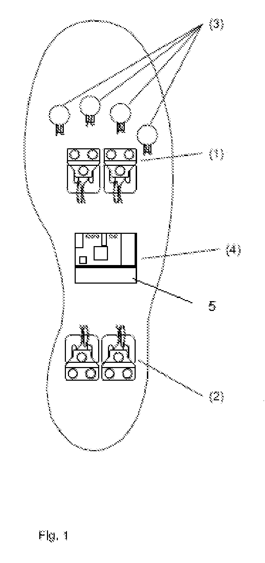

Figure 1 shows the footwear item used in the system to assist in balancing body weight and comprising:
- means for detecting the weight bearing on the front part of the foot and on the rear part of the foot, which in one possible embodiment includes one or more frontal load sensors (1) placed in the front part of the foot, and one or more rear load sensors (2) placed in the heel area.
- A printed circuit board (4) for controlling and transmitting / receiving signals.
- A lithium battery (5) for powering the elements installed in the footwear item.
- vibrating motors (3), positioned in the front part of the foot in front of the front sensor or sensors (1), with the number of vibrating motors (3) in one possible embodiment being four.

The printed circuit board (4) is responsible for receiving the load signal measured by the sensors (1) and (2), and is designed in such a way that when the ratio between the weight bearing on the front part and the weight bearing on the rear part exceeds a given threshold of imbalance, for example 70%, it issues an alarm signal that will be sent via wireless, for example via Bluetooth, to a mobile terminal or smartphone with an application especially designed, which, on the user's acceptance, begins an exercise of postural re-education, directing the user's attention to the body, as well as stimulating the front part of the sole of the foot in order to assist in correcting imbalance in the distribution of the body weight between the front and rear parts of the foot.

The stimulation exercise consists of sequential activation of the vibrating motors (3), which are positioned in the front part of the footwear item.

The operating mode of the system may be selected from the application installed in the smartphone, the possibilities being a mode with activation by pre-set thresholds or a mode with continuous transmission.

In the mode of activation by pre-set thresholds, in one possible embodiment specific load thresholds are established for the front sensor and for the rear sensor. The application sends an alarm to the user informing him/her of these levels, and gives him/her the possibility of performing the exercise or postponing it.

In continuous mode, the application will send the data to the device and from that moment on the vibrating motors will vibrate automatically when said threshold in the corresponding sensor is exceeded.

Having sufficiently described the nature of the present invention, and the means of implementing it, it is noted that within the same essence, it may be made in other embodiments differing in detail from that indicated herein as an example, and to which the protection obtained shall equally extend, provided that it does not alter, change or modify its basic principle.

## Claims

1. System for assisting in balancing body weight **characterised in that** it contains:
- a footwear item which in turn contains:
- Means for detecting the weight bearing on the front part of the foot and on the rear part of the foot,
- A printed circuit board (4) for control and transmission / reception of signals.
- A lithium battery (5) to power the elements installed in the footwear item.
- Vibrating motors (3),
- and a smartphone or similar in communication with the printed circuit board (4) of the footwear item, on which is installed an application with different graphs showing the level of body weight, guidelines and exercises.

2. System for assisting in balancing body weight, according to Claim 1 **characterised in that** the means of detection of the weight bearing on the front part of the foot and on the rear part of the foot comprises two front load sensors (1) placed in the front part of the sole of the foot, and two rear load sensors (2) placed in the area of the heel.

3. System for assisting in balancing body weight, according to Claim 1 **characterised in that** the vibrating motors (3) are positioned in the front part of the foot.

4. System for assisting in balancing body weight, according to Claim 1 **characterised in that** the vibrating motors (3) are arranged in the front part of the foot in front of the front load sensor.

5. System for assisting in balancing body weight, according to Claim 1 or 3 **characterised in that** the number of vibrating motors (3) is four.

6. System for assisting in balancing body weight, according to Claim 1 **characterised in that** the printed circuit board (4) is in charge of receiving the signal of the load value measured by the sensors (1) and (2), and is designed in such a way that when the ratio between the weight borne by the front part and the weight borne by the rear part exceeds a given threshold of imbalance, it issues an alarm signal to the smartphone fitted with an application to record and display the degrees of lateral and frontal weight imbalance, prepares the user for the exercises and activates operation of the vibrating motors (3).

7. System for assisting in balancing body weight, according to Claim 6 **characterised in that** the threshold of imbalance between the weight of the front and rear part from which an alarm is generated determined by the user, has options from 60 to 70% of the weight bearing on the rear area of the body.
